# EUROPÄISCHE PATENTSCHRIFT

(11) **EP 3 755 412 B1**
(45) Veröffentlichungstag und Bekanntmachung des Hinweises auf die Patenterteilung: **23.02.2022**
(21) Anmeldenummer: 19703023.2
(22) Anmeldetag: 24.01.2019
(51) Int. Cl.: A61M 25/01, A61M 1/28, A61M 1/36, A61M 25/00

(54) **KATHETERSYSTEM**
CATHETER SYSTEM
SYSTÈME DE CATHÉTER

(30) Priorität: 20.02.2018 DE 102018103742
(43) Veröffentlichungstag der Anmeldung: 30.12.2020
(73) Patentinhaber: Joline GmbH & Co. KG, 72379 Hechingen (DE)
(72) Erfinder: SEIDENBERGER, Dieter, 72379 Hechingen (DE)
(74) Vertreter: DREISS Patentanwälte PartG mbB
(86) Internationale Anmeldenummer: PCT/EP2019/051691
(87) Internationale Veröffentlichungsnummer: WO 2019/162028

(56) Entgegenhaltungen:
- US-A1- 2004 167 463
- US-A1- 2006 058 737
- US-A1- 2007 106 206
- US-A1- 2016 008 573

## Beschreibung

Die Erfindung betrifft ein Kathetersystem zum Einführen und Positionieren eines zweilumigen Katheters, insbesondere eines Dialysekatheters, in einem Blutgefäß. Das Kathetersystem umfasst einen zweilumigen Katheter mit einer distalen Katheterspitze, mit einem ersten Lumen und einem zweiten Lumen. Ferner umfasst das Kathetersystem einen sich durch das erste Lumen erstreckenden ersten Mandrin und einem sich durch das zweite Lumen erstreckenden zweiten Mandrin.

Derartige Kathetersysteme sind aus dem Stand der Technik vorbekannt. Mandrins (auch Stylets oder Stillets genannt) sind Hilfsmittel zum Einführen von Kathetern in Blutgefäße. Bei zweilumigen Kathetern, wie sie insbesondere bei der Dialyse verwendet werden, werden hierbei durch beide Lumen hindurch Mandrins eingeführt. Mandrins dienen hierbei einerseits zur Stabilisierung des Katheters gegen ein Ausknicken und somit zur Ermöglichung einer sicheren Einführung des Katheters in ein Blutgefäß. Ferner verhindern Mandrins in den Lumen ein Eindringen von Luft in das Blutgefäß sowie ein unerwünschtes Austreten und/oder Eindringen von Blut aus dem bzw. in den Katheter beim Einführen in das Blutgefäß. In der US 2016/0008573 A1 sind Kathetersysteme offenbart, bei welchem zwei separate Mandrins in distaler Richtung hinter dem distalen Ende der Katheterspitze zusammenführen. In US 2006/0058737 A1 ist ein Mandrin offenbart, der radioopak ausgeführt ist.

Aus der US 2011/0144620 A1 ist ein Kathetersystem vorbekannt. Dabei ist ein als Ring ausgebildeter Röntgenmarker proximal zur proximalsten Öffnung im distalen Endbereich des zweilumigen Katheters vorgesehen. Dieser Röntgenmarker kann ein Ring aus Edelmetall oder ein Aufdruck mit wolframhaltiger Drucktinte sein. Diese Röntgenmarker dienen dazu, den Katheter richtig im Blutgefäß zu implantieren. Die gewünschte Position kann beispielsweise am Übergang von der Superior-Vena-Cava (SVC) zur rechten Herzkammer (right atrium (RA)) liegen. Liegt der Röntgenmarker folglich an dieser Position, so ragt der funktionale Bereich des Katheters in die rechte Herzkammer. Der funktionale Bereich wird hierbei repräsentiert als der Bereich zwischen der distalen Katheterspitze und der proximalsten seitlichen Öffnung.

Die Verwendung eines solchen radioopaken Röntgenmarkers, der von einem Röntgengerät erkannt werden kann, kann nachteilig sein, da das radioopake Material am Körper anliegt und Körperkontakt hat. Ferner können solche üblichen Röntgenmarker nur kleine Bereiche sichtbar machen und zwar den Bereich des Röntgenmarkerrings. Die verwendeten radioopaken Materialien können insbesondere wenig körperfreundlich sein. Insbesondere bei Langzeitkathetern, die über mehrere Tage, Wochen oder sogar Monate im Körper einer Person verbleiben, kann der dauerhafte Kontakt der Röntgenmaterialien mit dem menschlichen Körper nachteilig sein.

Unter dem Begriff der Dialyse werden im Folgenden sämtliche Blutreinigungsverfahren verstanden, insbesondere die Hämodialyse, Hämofiltration, Hämodiafiltration, Peritonealdialyse, sowie Hämoperfusion.

Der Erfindung liegt insgesamt die Aufgabe zu Grunde, ein Kathetersystem bereitzustellen, welches körperverträglicher ist, und insbesondere kontrastreich und gut sichtbar ist.

Diese Aufgabe wird durch ein Kathetersystem mit den Merkmalen des Anspruchs 1 gelöst. Es ist demnach vorgesehen, dass beide Mandrins des Katheters radioopak ausgebildet sind, wobei das distale Ende des zweiten Mandrins in einem in proximaler Richtung liegenden Abstand von der distalen Katheterspitze angeordnet ist. Dabei kann der Abstand zwischen der distalen Katheterspitze und dem distalen Ende des zweiten Mandrins insbesondere einer funktionellen Katheterspitzenlänge entsprechen.

Dadurch, dass die Mandrins radioopak sind, können diese von einem Röntgengerät erkannt werden. Dies wird zur richtigen Positionierung des Katheters ausgenutzt und zwar dadurch, dass der zweite Mandrin zur distalen Katheterspitze beabstandet angeordnet ist. Wenn das distale Ende des zweiten Mandrins an der Bestimmungsposition detektiert worden ist, so ist der Katheter richtig positioniert. Die Bestimmungsposition kann beispielsweise das rechte Atrium sein. Von dieser Bestimmungsposition aus ragt die Katheterspitze sodann weiter in distaler Richtung in das Blutgefäß. Dieser Bereich zwischen dem distalen Ende des zweiten Mandrins und der distalen Katheterspitze kann insbesondere einer funktionellen Katheterspitzenlänge entsprechen, welche in das Blutgefäß, beispielsweise in das rechte Atrium, von der Bestimmungsposition ausgehend ragt. Dabei kann das distale Ende des zweiten Mandrins beispielsweise an der proximalsten Öffnung des zweiten Lumens liegen. Ausgehend von dieser proximalsten Öffnung in distaler Richtung liegt folglich der funktionelle Abschnitt. Dabei kann der erste Mandrin des ersten Lumens an der distalen Katheterspitze liegen, oder diese sogar überragen, so dass ein Ausknicken der Katheterspitze insbesondere beim Einführen des Kathetersystems in ein Blutgefäß verhindert werden kann.

Das zweite Lumen weist dabei eine distale Öffnung auf, wobei in proximaler Richtung wenigstens eine Seitenöffnung vorhanden ist, und wobei das distale Ende des zweiten Mandrins in proximaler Richtung hinter einem proximalen Ende der proximalsten Seitenöffnung liegt. Alternativ kann das distale Ende des zweiten Mandrins am proximalen Ende der proximalsten Seitenöffnung liegen. Folglich kann insbesondere die Distanz zwischen der distalen Katheterspitze und dem distalen Ende des zweiten Mandrins den funktionellen Katheterspitzenabschnitt darstellen. Seitenöffnungen dienen insbesondere dazu, dass dann, wenn die distale Lumenöffnung verschlossen ist, beispielsweise durch Anliegen an einem Blutgefäß, dennoch Blut durch den Katheter eingeführt oder entnommen werden kann.

Durch die Erfindung kann ein an der Außenseite des Katheters vorgesehener Röntgenmarker, welcher schädliche Auswirkungen auf den menschlichen Körper haben kann, vollständig entfallen. Stattdessen werden die Mandrins zur Katheterpositionierung verwendet. Diese werden nach der Positionierung des Katheters zur Benutzung des Katheters, beispielsweise für die Dialyse, entfernt. Insgesamt kann ein für den Körper verträglicheres Kathetersystem bereitgestellt werden. Ferner kann eine Abbildung der gesamten distalen Kathetherspitzengeometrie und -lage erhalten werden.

Eine vorteilhafte Weiterbildung der Erfindung sieht vor, dass der Abstand zwischen der Katheterspitze und dem distalen Ende des zweiten Mandrins zwischen 15 bis 50 mm, insbesondere zwischen 15 bis 30, weiter insbesondere 20 mm, beträgt. Dieser Bereich kann insbesondere der funktionellen Katheterspitzenlänge entsprechen und insbesondere den Bereich zwischen der proximalsten Öffnung des zweiten Lumens und der distalen Spitze des Katheters repräsentieren.

Eine weitere vorteilhafte Ausgestaltung der Erfindung sieht vor, dass das distale Ende des zweiten Mandrins innerhalb des zweiten Lumens liegt. Folglich kann das distale Ende des zweiten Mandrins insbesondere auch von einer distalen Öffnung des zweiten Lumens beabstandet sein. Das erste Lumen kann insbesondere dem venösen Lumen eines Dialysekatheters entsprechen, so dass das erste Mandrin die venöse Mandrin ist. Durch dieses venöse Lumen kann im späteren Einsatz des Katheters gereinigtes Blut dem Blutgefäß zugeführt werden. Dementsprechend kann das zweite Lumen das arterielle Lumen sein und das zweite Mandrin die arterielle Mandrin sein. Durch dieses arterielle Lumen kann Blut aus dem Blutgefäß entnommen werden und dem Dialysator zur Reinigung zugeführt werden.

Besonders bevorzugt ist, wenn der erste Mandrin zum Einführen des Katheters die Katheterspitze in distaler Richtung überragt. Der erste Mandrin kann folglich als Führungsmandrin dienen, so dass der Katheter sicher platziert werden kann. Dadurch, dass auch der erste Mandrin radioopak ist, kann die Position des ersten Mandrins bestimmt werden und insbesondere dessen distales Ende mittels eines Röntgengeräts visualisiert werden, um insbesondere ein Verletzen von Blutgefäßen beim Einführen des Katheters zu verhindern.

Besonders bevorzugt ist dabei, wenn der erste Mandrin und/oder der zweite Mandrin einen BaS04-Anteil zwischen 15-30 %wt, insbesondere zwischen 20 und 25 wt%, weiter insbesondere 24,5 wt%, aufweist bzw. aufweisen. Ein solcher Bariumsulfat-Anteil erlaubt eine Bereitstellung einer Röntgenopazität, sodass das Material im Röntgenkontrast sichtbar ist. Die Mandrins können insbesondere als Profilschläuche aufgebaut sein. Die Mandrins können neben dem genannten BaS04-Anteil eine Mischung (Compound) aus High-density Polyethylen (HDPE) und Low-density Polyethylen (LDPE) umfassen. Beispielsweise kann der HDPE-Anteil bei ca. 34 wt% liegen, während der LDPE-Anteil bei ca. 39 wt% liegen kann.

Der zweilumige Katheter kann aus einem Polyurethan (PUR) bestehen, beispielweise aus einem thermoplastischen Polyurethan-Elastomer (TPU). Der Katheter kann ebenfalls einen BaS04-Anteil enthalten. Denkbar wären 20wt% BaS04. Der Katheter kann eine Shore-Härte 85A aufweisen.

Besonders bevorzugt ist weiter, wenn das erste Lumen eine distale Öffnung umfasst, wobei der die Öffnung umgebende Bereich des Katheters die Katheterspitze umfasst. Die distale Öffnung des ersten Lumens kann folglich in distaler Richtung hinter der distalen Öffnung des zweiten Lumens liegen, die distale Öffnung des zweiten Lumens liegt folglich proximal zur distalen Öffnung des ersten Lumens. Eine derartige Ausgestaltung des Katheters kann insbesondere bei Dialysekathetern ein optimales Entnehmen von Blut und ein optimales Einführen von gereinigtem Blut in das Blutgefäß ermöglichen.

Vorzugsweise weist der Katheter eine Mittelwandung auf, die das erste Lumen vom zweiten Lumen trennt, und wobei das distale Ende der Mittelwandung die Katheterspitze darstellt. Ein solcher Katheter ist besonders einfach aufgebaut. Er kann folglich insbesondere zwei parallel zueinander verlaufende, jeweils wenigstens ein Lumen bildende Schlauchabschnitte aufweisen, wobei zwischen den beiden Schlauchabschnitten die Mittelwandung liegt.

Eine besonders bevorzugte Weiterbildung der Erfindung ergibt sich daraus, dass der Katheter als Dialysekatheter ausgebildet ist, wobei das erste Lumen das venöse Lumen ist, und wobei das zweite Lumen das arterielle Lumen ist.

Vorzugsweise ist der Katheter zur Langzeitimplantation ausgebildet. Ein solcher Katheter kann insbesondere mehrere Tage, mehrere Wochen oder mehrere Monate oder bis zu einem Jahr im Patienten verbleiben. Bei solchen Langzeitanwendungen ist die Erfindung besonders vorteilhaft, da ein Röntgenmarker, der beispielsweise Wolfram enthalten kann, und in Körperkontakt ist, entfallen kann und stattdessen eine Positionierung des Katheters über die Mandrins erfolgt. Ein solcher Katheter kann insbesondere im proximalen Bereich Fixiermittel aufweisen, um die Katheterposition zu fixieren.

Weitere Einzelheiten und vorteilhafte Ausgestaltungen der Erfindung sind der folgenden Beschreibung zu entnehmen, anhand derer die in den Figuren dargestellte Ausführungsform der Erfindung näher beschrieben und erläutert ist. Es zeigen:

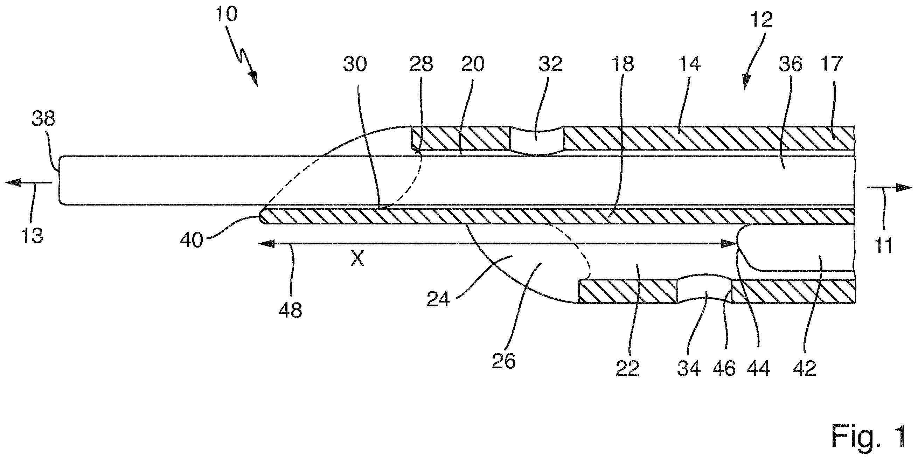

- Figur 1: eine schematische Längsschnittdarstellung des distalen Endbereichs eines Kathetersystems gemäß einer Ausführungsform.

Figur 1 zeigt insgesamt den distalen Endbereich 12 eines Kathetersystems 10, umfassend zunächst einen als Dialysekatheter ausgebildeten Katheter 14, der zum Einführen in ein Blutgefäß ausgebildet ist.

Der proximale Bereich des Kathetersystems 10 ist nicht gezeigt. Der nicht gezeigte proximale Bereich des Katheters 14 ist dabei zum Anschluss an einen Dialysator über ein Blutschlauchsystem ausgebildet, um entnommenes Blut zu reinigen und gereinigtes Blut einem Patienten zuzuführen. Der proximale Endbereich des Katheters 10 kann insbesondere wie in einer der in der Patentanmeldung DE 10 2017 118 820.7 offenbarten Ausführungsformen ausgebildet sein, deren Offenbarungsgehalt durch Verweis vollständig mit in vorliegende Patentanmeldung einbezogen wird.

Der Katheter 14 weist im Querschnitt eine runde Kontur mit einer Außenwandung 17 auf. Ferner weist der Katheter 14 eine sich entlang einer Längsachse erstreckende, eine Mittelebene bildende Mittelwandung 18 auf. Die Mittelwandung 18 trennt dabei das Innere des Katheters in ein erstes, als Einleitlumen (venöses Lumen) ausgebildetes, Lumen 20 und ein zweites, als Entnahmelumen (arterielles Lumen) ausgebildetes, Lumen 22 auf. Durch das Entnahmelumen 22 kann einem Patienten Blut entnommen werden, im Dialysator gereinigt werden und das so gereinigte Blut dem Patienten sodann durch das Einleitlumen 20 wieder zugeführt werden. An seinem distalen Ende 24 weist das Entnahmelumen 22 eine als Entnahmeöffnung ausgebildete distale Öffnung 26 auf. Das Einleitlumen 20 weist an seinem distalen Ende 28 eine als Einleitöffnung ausgebildete distale Öffnung 30 auf.

Wie aus Figuren 1 weiter hervorgeht, weist das Einleitlumen 20 von der Einleitöffnung 30 in proximaler Richtung 11 beabstandet eine venöse Seitenöffnung 32 auf. Diese Seitenöffnung 32 dient dazu, dass Blut auch dann eingeleitet werden kann, falls die Einleitöffnung 30 verschlossen sein sollte, beispielsweise durch Anliegen an einem Blutgefäß. Entsprechend ist, wie aus Figur 2 deutlich wird, am Entnahmelumen 22 eine von der Entnahmeöffnung 26 in proximaler Richtung 11 beabstandete arterielle Seitenöffnung 34 vorgesehen sodass Blut auch dann entnommen werden kann, falls die Entnahmeöffnung 26 verschlossen sein sollte, beispielsweise durch Anliegen an einem Blutgefäß. Die Einleitöffnung 28 liegt folglich in distaler Richtung 13 hinter der Entnahmeöffnung 26, so dass die Einleitöffnung 28 distaler als die Entnahmeöffnung 26 ist.

Die genaue Geometrie des in Figur 1 schematisch gezeigten distalen Endbereichs des Katheters 14 kann insbesondere wie in einer der in der Patentanmeldung DE 10 2017 118 819.3 offenbarten Ausführungsformen ausgebildet sein, deren Offenbarungsgehalt durch Verweis vollständig mit in vorliegende Patentanmeldung einbezogen wird.

Im Einleitlumen 20 ist ein erstes Mandrin 36 angeordnet. Dieses erstreckt sich folglich durch das Einleitlumen 20. Hierbei handelt es sich um das venöse Mandrin. Das distale Ende 38 des ersten Mandrins 36 ragt dabei in distaler Richtung 13 über das distale Ende 40 des Katheters 14 hinaus. Das distale Ende 38 bildet folglich das distale Ende des Kathetersystems 10.

Ebenso ist im Entnahmelumen 22 ein zweiter Mandrin 42 angeordnet. Das distale Ende 44 des zweiten Mandrins 42 endet dabei am proximalen Ende 46 der arteriellen Seitenöffnung 34. Der Abstand X zwischen dem distalen Ende 44 und der Katheterspitze 40 stellt den funktionalen Katheterspitzenabschnitt 48 dar, welcher folglich vom proximalen Ende 46 der Seitenöffnung 34 bis zur Katheterspitze 40 reicht. Dieser Abstand X kann beispielsweise 20 mm betragen.

Beide Mandrins 36 und 42 sind radioopak ausgebildet. Dabei enthalten diese beispielsweise 24% Bariumsulfat (BaS04), sodass die Mandrins aus im Röntgenkontrast sichtbarem Material bestehen und von einem Röntgengerät erkannt werden können. Somit kann beim Einführen des Katheters in ein Blutgefäß zunächst die Position, insbesondere das distale Ende 38 des ersten Mandrins 36 ermittelt werden, um so den Katheter in das Blutgefäß in distaler Richtung 13 seiner Bestimmungsposition zuzuführen.

Die Bestimmungsposition kann beispielsweise am Übergang zwischen der superior vena cava (SVC) und der rechten Herzkammer (right atrium (RA)) liegen. Diese Bestimmungsposition kann hierbei insbesondere dann erreicht sein, wenn das distale Ende 44 des zweiten Mandrins 42 an dieser Position angelangt ist. Der funktionelle Abschnitt 48 des Katheters 14 ragt folglich ausgehend von dieser Bestimmungsposition in distaler Richtung 13 in das Blutgefäß, beispielsweise das rechte Atrium, weiter. Der Chirurg kann folglich insbesondere feststellen, wo das proximale Ende 46 der proximalsten Seitenöffnung 34 im Entnahmelumen 22 liegt. Nach der Positionierung werden die Mandrins 36 und 42 entnommen. Sodann kann eine Dialyse durchgeführt werden, wobei Blut aus dem Blutgefäß durch das Entnahmelumen 22 entnommen wird, einem Dialysator zur Reinigung zugeführt wird und das gereinigte Blut über das Einleitlumen 20 dem Blutgefäß wieder zugeführt werden. Der Katheter 14 kann insbesondere ein Langzeitkatheter sein, der insbesondere mehrere Tage, Wochen oder Monate oder bis zu einem Jahr im Körper verbleiben kann.

Im Gegensatz zum Stand der Technik kann ein insbesondere wolframhaltiger Röntgenmarker an der Außenseite des Katheters im Bereich des proximalen Endes 46 der proximalsten Seitenöffnung 34 vollständig entfallen. Lediglich der Katheter 14 verbleibt am Körper in Körperkontakt.

## Patentansprüche

1. Kathetersystem (10) zum Einführen und Positionieren eines zweilumigen Katheters (14), insbesondere eines Dialysekatheters, in einem Blutgefäß, umfassend einen zweilumigen Katheter (14) mit einer distalen Katheterspitze (40), mit einem ersten Lumen (20) und einem zweiten Lumen (22), einem sich durch das erste Lumen (20) erstreckenden ersten Mandrin (36) und einem sich durch das zweite Lumen (22) erstreckenden zweiten Mandrin (42), wobei das zweite Lumen (22) eine distale Öffnung (26) und in proximaler Richtung wenigstens eine Seitenöffnung (34) aufweist, **dadurch gekennzeichnet, dass** beide Mandrins (36, 42) des Katheters (14) radioopak ausgebildet sind, wobei der Katheter (14) zum Einführen in das Blutgefäß so ausgebildet ist, dass das distale Ende (44) des zweiten Mandrins (42) in proximaler Richtung (11) hinter oder an einem proximalen Ende (46) der proximalsten Seitenöffnung (34) liegt und in einem in proximaler Richtung (11) liegenden Abstand (X) von der distalen Katheterspitze (40) angeordnet ist, wobei der Abstand (X) zwischen der distalen Katheterspitze (40) und dem distalen Ende (44) des zweiten Mandrins (42) einen funktionellen Katheterspitzenabschnitt (48) darstellt.

2. Kathetersystem (10) nach Anspruch 1, wobei der Abstand (X) zwischen der distalen Katheterspitze (40) und dem distalen Ende (44) des zweiten Mandrins (42) zwischen 15 bis 50 mm, insbesondere zwischen 15 bis 30, weiter insbesondere 20 mm, beträgt.

3. Kathetersystem (10) nach Anspruch 1 oder 2, wobei das distale Ende (44) des zweiten Mandrins (42) innerhalb des zweiten Lumens (22) liegt.

4. Kathetersystem (10) nach einem der vorhergehenden Ansprüche, wobei der erste Mandrin (36) zum Einführen des Katheters (14) die Katheterspitze (40) in distaler Richtung (13) überragt.

5. Kathetersystem (10) nach einem der vorhergehenden Ansprüche, wobei der erste Mandrin (36) und/oder der zweite Mandrin (42) einen BaSO4-Anteil zwischen 15-30 %wt, insbesondere zwischen 20 und 25 wt%, weiter insbesondere 24 wt%, aufweist bzw. aufweisen.

6. Kathetersystem (10) nach einem der vorhergehenden Ansprüche, wobei das erste Lumen (20) eine distale Öffnung (30) umfasst, und wobei der die Öffnung umgebende Bereich des Katheters (14) die Katheterspitze (40) umfasst.

7. Kathetersystem (10) nach einem der vorhergehenden Ansprüche, wobei der Katheter (14) eine Mittelwandung (18) aufweist, die das erste Lumen (20) vom zweiten Lumen (22) trennt, und wobei das distale Ende der Mittelwandung (18) die Katheterspitze (40) darstellt.

8. Kathetersystem (10) nach einem der vorhergehenden Ansprüche, wobei der Katheter (14) als Dialysekatheter ausgebildet ist, wobei das erste Lumen (20) das venöse Lumen ist, und wobei das zweite Lumen (22) das arterielle Lumen ist.

## Claims

1. Catheter system (10) for inserting and positioning a double-lumen catheter (14), in particular a dialysis catheter, in a blood vessel, comprising a double-lumen catheter (14) with a distal catheter tip (40), with a first (20) and second lumen (22), an initial mandrin (36) throughout the length of the first lumen (20) and a second mandrin (42) throughout the length of the second lumen (22), whereby the second lumen (22) comprises a distal opening (26) and at least one proximal side opening (34), **characterised in that** both mandrins (36, 42) of the catheter (14) are radiopaque, whereby the catheter (14) is designed for insertion into the blood vessel such that the distal end (44) of the second mandrin (42) is located proximally (11) behind or at a proximal end (46) of the most proximal side opening (34) and at a proximal (11) distance (X) from the distal catheter tip (40), whereby the distance (X) between the distal catheter tip (40) and the distal end (44) of the second mandrin (42) forms a functional catheter tip segment (48).

2. Catheter system (10) as per claim 1, whereby the distance (X) between the distal catheter tip (40) and the distal end (44) of the second mandrin (42) is between 15 and 50 mm, more specifically between 15 and 30 mm, yet more specifically 20 mm.

3. Catheter system (10) as per claim 1 or 2, whereby the distal end (44) of the second mandrin (42) is within the second lumen (22).

4. Catheter system (10) as per one of the preceding claims, whereby the first mandrin (36) for inserting the catheter (14) distally (13) surpasses the catheter tip (40).

5. Catheter system (10) as per one of the preceding claims, whereby the first mandrin (36) and/or the second mandrin (42) comprise(s) a BaSO4 content between 15-30 wt%, more specifically between 20 and 25 wt%, yet more specifically 24 wt%.

6. Catheter system (10) as per one of the preceding claims, whereby the first lumen (20) comprises a distal opening (30), and whereby the area of the catheter (14) surrounding the opening comprises the catheter tip (40).

7. Catheter system (10) as per one of the preceding claims, whereby the catheter (14) comprises a middle partition (18) that separates the first lumen (20) from the second lumen (22), and whereby the distal end of the middle partition (18) forms the catheter tip (40).

8. Catheter system (10) as per one of the preceding claims, whereby the catheter (14) is a dialysis catheter, whereby the first lumen (20) is the venous lumen, and whereby the second lumen (22) is the arterial lumen.

## Revendications

1. Système de cathéter (10) pour l'introduction et le positionnement d'un cathéter à double lumière (14), en particulier d'un cathéter de dialyse, dans un vaisseau sanguin, comprenant un cathéter à double lumière (14) avec une pointe de cathéter distale (40), avec une première lumière (20) et une deuxième lumière (22), un premier mandrin (36) s'étendant à travers la première lumière (20) et un deuxième mandrin (42) s'étendant à travers la deuxième lumière (22), dans lequel la deuxième lumière (22) présente une ouverture distale (26) et dans la direction proximale au moins une ouverture latérale (34), **caractérisé en ce que** les deux mandrins (36, 42) du cathéter (14) sont réalisés de manière radio-opaque, dans lequel le cathéter (14) est réalisé pour être introduit dans le vaisseau sanguin, de sorte que l'extrémité distale (44) du deuxième mandrin (42) se situe dans la direction proximale (11) derrière ou à une extrémité proximale (46) de l'ouverture latérale la plus proximale (34) et est disposée à une distance (X) située dans la direction proximale (11) de la pointe de cathéter distale (40),
dans lequel la distance (X) entre la pointe de cathéter distale (40) et l'extrémité distale (44) du deuxième mandrin (42) représente une partie de pointe de cathéter fonctionnelle (48) .

2. Système de cathéter (10) selon la revendication 1, dans lequel la distance (X) entre la pointe de cathéter distale (40) et l'extrémité distale (44) du deuxième mandrin (42) est comprise entre 15 et 50 mm, en particulier entre 15 et 30, plus particulièrement atteint 20 mm.

3. Système de cathéter (10) selon la revendication 1 ou 2, dans lequel l'extrémité distale (44) du deuxième mandrin (42) se situe à l'intérieur de la deuxième lumière (22).

4. Système de cathéter (10) selon l'une quelconque des revendications précédentes, dans lequel le premier mandrin (36) pour introduire le cathéter (14) fait saillie de la pointe de cathéter (40) dans la direction distale (13).

5. Système de cathéter (10) selon l'une quelconque des revendications précédentes, dans lequel le premier mandrin (36) et/ou le deuxième mandrin (42) présente ou présentent une part de BaSO4 comprise entre 15-30 % en poids, en particulier entre 20 et 25 % en poids, plus particulièrement de 24 % en poids.

6. Système de cathéter (10) selon l'une quelconque des revendications précédentes, dans lequel la première lumière (20) comprend une ouverture distale (30), et dans lequel la zone du cathéter (14) entourant l'ouverture comprend la pointe de cathéter (40).

7. Système de cathéter (10) selon l'une quelconque des revendications précédentes, dans lequel le cathéter (14) présente une paroi centrale (18), qui sépare la première lumière (20) de la deuxième lumière (22), et dans lequel l'extrémité distale de la paroi centrale (18) représente la pointe de cathéter (40).

8. Système de cathéter (10) selon l'une quelconque des revendications précédentes, dans lequel le cathéter (14) est réalisé sous la forme d'un cathéter de dialyse, dans lequel la première lumière (20) est la lumière veineuse, et dans lequel la deuxième lumière (22) est la lumière artérielle.
